# EUROPEAN PATENT SPECIFICATION

(11) **EP 1 711 140 B1**
(45) Date of publication and mention of the grant of the patent: **06.03.2013**
(21) Application number: 05705392.8
(22) Date of filing: 10.01.2005
(51) Int. Cl.: A61F 2/44

(54) **SPINAL ARTHROPLASTY DEVICE AND METHOD**
WIRBELSÄULENARTHROPLASTIEVORRICHTUNG UND VERFAHREN
DISPOSITIF ARTHROPLASTIQUE VERTEBRAL ET METHODE ASSOCIEE

(30) Priority: 09.01.2004 US 534960 P
(43) Date of publication of application: 18.10.2006
(73) Proprietor: Warsaw Orthopedic, Inc., Warsaw, IN 46581 (US)
(72) Inventor: HUMPHREYS, Steven, C., Challanooga, TN 37421 (US); HODGES, Scott, D., Oottewah, TN 37363 (US)
(74) Representative: Viering, Jentschura & Partner
(86) International application number: PCT/US2005/000706
(87) International publication number: WO 2005/070354

(56) References cited:
- WO-A-00/04851
- DE-U1-202004 015 198
- FR-A- 2 799 638
- US-A- 6 113 637
- US-A1- 2003 004 572
- US-A1- 2003 055 427
- US-B1- 6 572 653

## Description

### TECHNICAL FIELD

Embodiments of the invention relate generally to devices for accomplishing spinal surgery, and more particularly in some embodiments, to spinal arthroplasty devices capable of being placed posteriorally into the vertebral disc space. Various implementations of the invention are envisioned, including use in total spine arthroplasty replacing, via a posterior approach, both the disc and facet functions of a natural spinal joint

### BACKGROUND

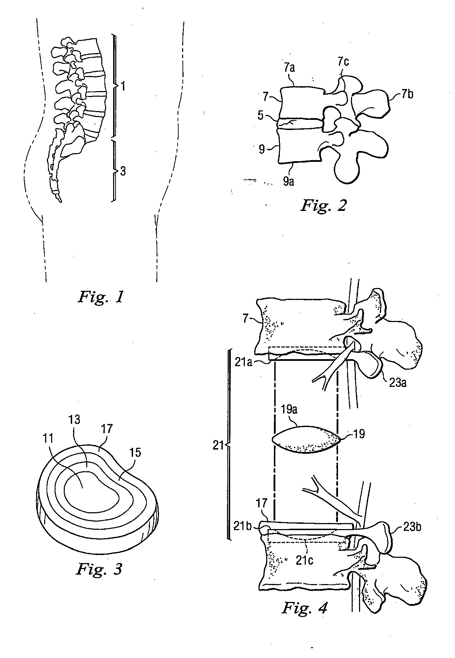

As is known the art, in the human anatomy, the spine is a generally flexible column that can take tensile and compressive loads, allows bending motion and provides a place of attachment for ribs, muscles and ligaments. Generally, the spine is divided into three sections: the cervical, the thoracic and the lumbar spine. Figure 1 illustrates schematically the lumbar spinal 1 and the sacrum regions 3 of a healthy, human spinal column. The sections of the spine are made up of individual bones called vertebrae and the vertebrae are separated by intervertebral discs which are situated therebetween.

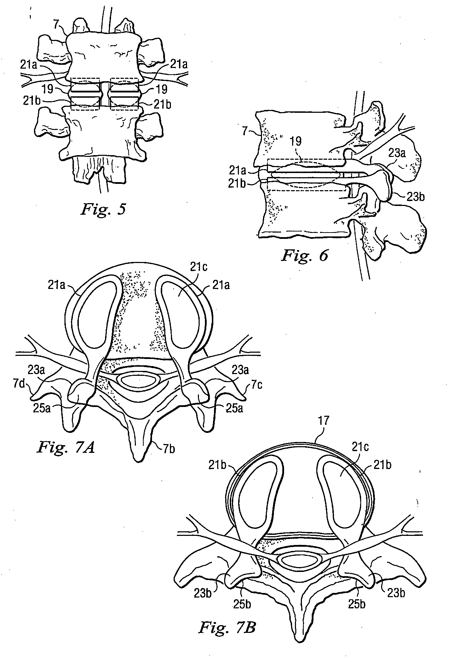

Figure 2 illustrates a portion of the right side of a lumbar spinal region with a healthy intervertebral disc 5 disposed between two adjacent vertebrae 7, 9. In any given joint, the top vertebra may be referred to as the superior vertebra and the bottom one as the inferior vertebra. Each vertebra comprises a generally cylindrical body 7a, 9a, which is the primary area of weight bearing, and three bony processes, e.g., 7b, 7c, 7d (two of which are visible in Figure 2). As shown in Figure 7A, in which all of the processes are visible, processes 7b, 7c, 7d extend outwardly from vertebrae body 7 at circumferentially spaced locations. The processes, among other functions, provide areas for muscle and ligament attachment. Neighboring vertebrae may move relative to each other via facet components 7e (Fig. 2), which extend from the cylindrical body of the vertebrae and are adapted to slide one over the other during bending to guide movement of the spine. There are two facet joints, each defined by upper and lower facet components, associated with adjacent vertebra. A healthy intervertebral disc is shown in Figure 3. As shown in Figure 3, an intervertebral disc has 4 regions: a nucleus pulposus 11, a transition zone 13, an inner annulus fibrosis region 15 and an outer annulus fibrosis 17. Generally, the inner annulus fibrosis region 15 and the outer annulus fibrosis region 17 are made up of layers of a fibrous gristly material firmly attached to the vertebral bodies above and below it. The nucleus pulposus 11 is typically more hydrated in nature.

These intervertebral discs function as shock absorbers and as joints. They are designed to absorb the compressive and tensile loads to which the spinal column may be subjected while at the same time allowing adjacent vertebral bodies to move relative to each other a limited amount, particularly during bending (flexure) of the spine. Thus, the intervertebral discs are under constant muscular and/or gravitational pressure and generally are the first parts of the lumbar spine to show signs of "wear and tear".

Facet joint degeneration is also common because the facet joints are in almost constant motion with the spine. In fact, facet joint degeneration and disc degeneration frequently occur together. Generally, although one may be the primary problem while the other is a secondary problem resulting from the altered mechanics of the spine, by the time surgical options are considered, both facet joint degeneration and disc degeneration typically have occurred. For example, the altered mechanics of the facet joints and/or intervertebral disc may cause spinal stenosis, degenerative spondylolisthesis, and degenerative scoliosis.

One surgical procedure for treating these conditions is spinal arthrodesis (i.e., spine fusion), which has been performed both anteriorally and/or posteriorally. The posterior procedures include in-situ fusion, posterior lateral instrumented fusion, transforaminal lumbar interbody fusion ("TLIF") and posterior lumbar interbody fusion ("PLIF"). Solidly fusing a spinal segment to eliminate any motion at that level may alleviate the immediate symptoms, but for some patients maintaining motion may be advantageous. It is also known to surgically replace a degenerative disc or facet joint with an artificial disc or an artificial facet joint, respectively. However, none of the known devices or methods provide the advantages of the embodiments of the present disclosure.

Accordingly, the foregoing shows there is a need for an improved spinal arthroplasty that avoids the drawbacks and disadvantages of the known implants and surgical techniques.

US 2003/0004572 A1 discloses a kit for spine joint replacement comprising an artificial disc and prosthesis for the replacement of at least a portion of the bone of a facet located on a mammalian vertebra. The prosthesis comprises a surface that articulates with another facet surface and a fixation portion that is implanted into an interior bone space of said vertebra. The surface and the fixation portion are connected to each other. The artificial disc and the prosthesis cooperate so as to restore the natural biomechanics of a spinal motion segment.

US 2003/0055427 A1 discloses an intervertebral stabilization device intended for joining two adjacent vertebrae. The intervertebral stabilization device comprises an implant intended to be inserted at least partially between the vertebral bodies of the two adjacent vertebrae. The implant is adapted to give said two adjacent vertebral bodies at least one mutual degree of freedom. The device also comprises at least one extra-discal member disposed to the rear of the intervertebral space, adapted to damp a displacement between said vertebrae at least in the intervertebral flexion direction.

WO 00/04851 discloses a disc prosthesis including a cylindrical housing. The housing includes an upper half housing and a lower half housing. An upper fixator wing is welded to the upper half housing and a lower fixator wing is welded to the lower half housing so as to stabilize the prosthesis to bone. A plurality of resilient, viscoelastic discs are interposed between the two half housings to maintain the housing halves separate from one another and to provide for a defined range of motion.

US 6 113 637 A discloses an intervertebral joint prostheses having flanges for the engagement with vertebrae.

FR 27 99 638 A discloses a vertebral fixator forming a discal prosthesis, wherein the discal prosthesis has substantially the height of an intervertebral disk to be replaced. The vertebral fixator comprises at least one essentially rigid first element intended to be fixed to the articular face of one of two adjacent vertebrae, and at least one essentially rigid second element fixed to the articular face of the other vertebrae. The essentially rigid first and second elements are connected by at least one intermediate element having articular mobility. The first and second elements and the intermediate element are shaped dimensionally for introduction into the intervertebral space via a posterior or lateral surgical route. The essentially rigid first and second elements have fixation members capable, once the fixator has been arranged in the intervertebral space, of protruding from the two rigid elements essentially at right angles to their surfaces in contact with the corresponding surface of the vertebral body, in order to penetrate the vertebral body.

US 6,572,653 B1 discloses a vertebral implant having two pairs of hydroxyapatite coated superior and inferior supports. Between these supports, there are allocated two springs.

### SUMMARY

According to the present invention, an artificial vertebral joint as claimed in claim 1 is provided. The subclaims show some examples for such an artificial vertebral joint.

A first embodiment of this disclosure provides an artificial spinal joint for creating at least a portion of a coupling between a superior vertebra and an inferior vertebra. The artificial spinal joint may include an anterior joint replacement component and a bridge coupled to the anterior joint replacement that extends posteriorly from the anterior joint replacement. Additionally, a posterior joint replacement component may be coupled to the bridge as part of the artificial spinal joint.

In another embodiment, the artificial spinal joint includes an anterior joint replacement component with a left upper member, a left lower member that articulates with the left upper member, a right upper member, and a right lower member that articulates with the right upper member. In this embodiment, the left lower member and the right lower member are each configured to connect to the other.

The embodiments disclosed may be useful for degenerative changes of the lumbar spine, post-traumatic, discogenic, facet pain or spondylolisthesis, and/or to maintain motion in multiple levels of the lumbar spine.

Additional and alternative features, advantages, uses and embodiments are set forth in or will be apparent from the following description, drawings, and claims.

### BRIEF DESCRIPTION OF THE DRAWINGS

Figure 1 is a side elevation schematic view of the lumbar spinal and the sacrum regions of a healthy, human spinal column.
Figure 2 is a detailed perspective view showing a portion of the right side of the lumbar vertebrae shown in Figure 1 with a healthy disc disposed between two vertebrae.
Figure 3 is a top perspective view of the intervertebral disc shown in Figure 2 illustrating the major portions of the disc.
Figure 4 is a side exploded elevation view of a portion of a lumbar spine showing a first embodiment of an artificial intervertebral joint constructed according to the principles of the disclosure.
Figure 5 is an anterior elevation view of a portion of a lumbar spine showing the superior, disc and inferior portions of the left and right halves of an assembled artificial intervertebral joint constructed according to the first embodiment of the disclosure.
Figure 6 is a side elevation view of the right half of the artificial intervertebral joint shown in Figure 5.
Figure 7A is a transverse, bottom-up-view of a portion of a lumbar spine showing the superior portion of the artificial intervertebral joint illustrated in Figure 4.
Figure 7B is a transverse, top-down-view of a portion of a lumbar spine showing the inferior portion of the artificial intervertebral joint illustrated in Figure 4.
Figure 8 is a transverse, bottom-up-view of a portion of a lumbar spine showing a second embodiment of a superior portion of an artificial intervertebral joint in which pedicle screws are used to assist in implantation.
Figure 9 is a transverse, top-down-view of a portion of a lumbar spine showing a second embodiment of an inferior portion of an artificial intervertebral joint in which pedicle screws are used to assist in implantation.
Figure 10 is a lateral view of a portion of a lumbar spine showing the superior portion of the artificial intervertebral joint shown in Figure 8 with one of the pedicle screws being visible.
Figure 11 is a lateral view of a portion of a lumbar spine showing the inferior and integrated disc portions of an artificial integral intervertebral joint shown in Figure 9 with one of the pedicle screws being visible.
Figure 12 is a posterior view of a portion of a lumbar spine showing the superior portion of the artificial intervertebral joint shown in Figure 8 with two pedicle screws being visible.
Figure 13 is a posterior view of a portion of a lumbar spine showing the inferior portion of the artificial intervertebral joint shown in Figure 9 with two pedicle screws being visible.
Figure 14 is a side elevation view of a portion of a lumbar spine showing the second embodiment with pedicle screws in an assembled position.
Figure 15 is a posterior view of a portion of a lumbar spine showing a third embodiment of the inferior, disc and superior portions of an artificial intervertebral joint in which tension bands are used.
Figure 16 is a side elevation view of a portion of a lumbar spine showing the third embodiment in which tension bands are used in an assembled position.
Figure 17 is a transverse, bottom-up-view of a portion of a lumbar spine showing the superior portion of a fourth embodiment of an artificial intervertebral joint constructed according to the principles of the disclosure in which the facet joints are not replaced.
Figure 18 is a transverse, top-down-view of a portion of a lumbar spine showing the inferior portion of the fourth embodiment of an artificial intervertebral joint.

### DESCRIPTION

The drawings illustrate various embodiments of an artificial intervertebral joint for replacing an intervertebral disc or the combination of an intervertebral disc and at least one corresponding facet joint. Various embodiments of the artificial intervertebral joint according to the principles of the disclosure may be used for treating any of the problems that lend themselves to joint replacement including particularly, for example, degenerative changes of the lumbar spine, post-traumatic, discogenic, facet pain or spondylolisthesis and/or to maintain motion in multiple levels of the lumbar spine.

Figures 4 - 7 illustrate a first exemplary embodiment of an artificial intervertebral joint. As illustrated in Figures 4 and 5, each joint is composed of two arthroplasty halves, each of which has a spacer or disc 19 and a retaining portion 21. The retaining portion 21 includes a first retaining portion 21a and a second retaining portion 21b. In the example illustrated in Figure 4, the first retaining portion 21 a is superior to (above) the second retaining portion 21b and the disc 19 is situated therebetween. Although the artificial intervertebral joint according to this exemplary embodiment has two halves for each of the first retaining portion and the second retaining portion, it should be understood that alternative embodiments may be implemented such that the artificial intervertebral joint has a single first retaining member, a single second retaining member and a single spacer. It should also be understood that alternative embodiments may also be carried out with arthroplasties having a first retaining portion, a second retaining portion, and/or a disc which each consist of unequal sized halves or more than two components.

Further, as illustrated in Figure 4, the first retaining portion 21a and the second retaining portion 21b are situated between two adjacent vertebrae. More particularly, the first retaining portion may be situated along an inferior surface of the upper of the two adjacent vertebrae and the second retaining portion may be situated above a superior surface of the lower of the two adjacent vertebrae. However, it should be understood by one of ordinary skill in the art that the first retaining portion and second retaining portion are not limited to such an arrangement, and may be oriented in different positions and/or shaped differently than what is illustrated herein.

The surfaces of the retaining portions 21 a, 21b of the arthroplasty that contact the remaining end plates of the vertebrae may be coated with a beaded material or plasma sprayed to promote bony ingrowth and a firm connection therebetween. In particular, the surface to promote bone ingrowth may be a cobalt chromium molybdenum alloy with a titanium/calcium/phosphate double coating, a mesh surface, or any other effective surface finish. Alternatively or in combination, an adhesive or cement such as polymethylmethacrylate (PMMA) may be used to fix all or a portion of the implants to one or both of the endplates.

As discussed in more detail below, a significant portion of the outer annulus region 17 (see, e.g., Figures 4, 7B), in some embodiments about 300 degrees, may be retained on the inferior portion of the end plate, which acts as a stop retaining the lower retaining portions in place until bone ingrowth occurs to firmly attach the retaining portions to their respective vertebrae (Figure 4 only shows a portion of the outer annulus 17 that is retained). In contrast, in conventional anterior arthroplasty about 270 degrees of the outer annulus region 17 typically is removed. In addition, pedicle screws may also be used for immediate fixation as described in more detail in connection with other embodiments discussed below.

In the various embodiments of this disclosure, the first retaining portion 21 a and the second retaining portion 21b are structured so as to retain the disc 19 therebetween. For example, in the case of a disc 19 with two convex surfaces 19a, each of the first retaining portion 21a and the second retaining portion 21b may have a concave surface 21c which defines a space within which the disc 19 may be retained. For example, in the exemplary embodiment shown in Figure 4, the upper convex surface 19a of the disc 19 fits within the concavity defmed by the concave surface 21c of the first retaining portion 21a and the lower convex surface 19b of the disc 19 fits within the concavity defined by the concave surface 21c of the second retaining portion 21b.

Figure 5 illustrates an anterior view of an exemplary assembled artificial intervertebral joint with both arthroplasty halves in place, and Figure 6 shows a side view of the assembled artificial intervertebral joint shown in Figure 5. As illustrated in Figures 5 and 6, the disc 19 is retained between the first retaining portion 21 a and the second retaining portion 21b. It should be understood that although the disc 19 may be held between the first retaining portion 21a and the second retaining portion 21b, the disc 19 is free to slidably move within the space defined by the corresponding surfaces 21a of the first retaining portion 21a and the second retaining portion 21b. In this manner, limited movement between the adjacent vertebrae is provided.

In the exemplary embodiment illustrated in Figures 4, 5 and 6, the disc 19 is a separate component which is inserted between the first retaining portion 21a and the second retaining portion 21b. However, as discussed below, it should be understood that the spacer or disc 19 may be integrally formed with or integrated into in one or both of the first retaining portion 21a and the second retaining portion 21b.

In the exemplary embodiment of the disclosure, as illustrated best in Figures 4, 6, 7A and 7B, each of the retaining portions of the artificial intervertebral joint includes a first artificial facet component 23a and a second artificial facet component 23b. As shown in Figures 7A and 7B, the first artificial facet component 23a has a face 25a and the corresponding second artificial facet component 23b has a face 25b configured such that the face 25a matingly fits with the face 25b to stabilize adjacent vertebrae while preserving and guiding the mobility of each vertebrae with respect to the other vertebrae. Each set of the upper and lower retaining portions 21 a, 21b may have a pair of facet components 23a, 23b, which together define a facet joint. For a total joint replacement with facets according to this embodiment, the left and right arthroplasties would define two adjacent facet joints when viewed from the posterior.

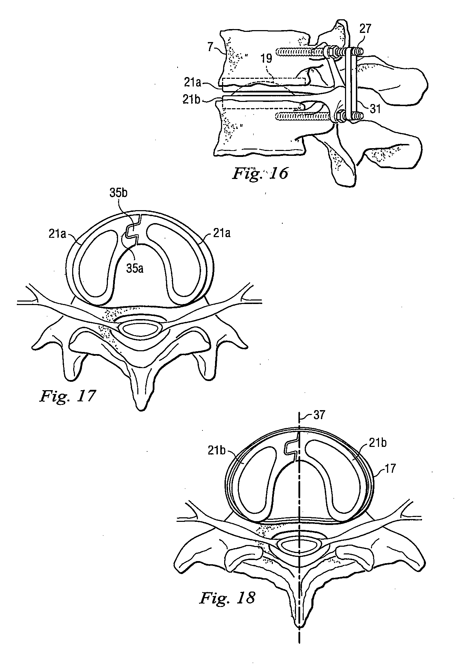

Regardless of whether artificial facet joints are provided, the respective upper and lower retaining portions associated with the left and right halves of the arthroplasty may be completely independent from the other. That is, as shown in Figure 7A, for example, the first retaining portions 21a associated with each half are not in direct contact with each other. The same is true with respect to the second retaining portions 21 b shown in Figure 7B. However, it should be understood by one of ordinary skill in the art that, even in the embodiment of the disclosure which includes artificial facet joints, at least a portion of the first retaining portions 21a of each half and/or at least a portion of the second retaining portions 21 b of each half may directly contact and/or be connected to each other as described in more detail in connection with the discussion of Figures 17-18.

Further, in the various embodiments of the disclosure, the disc 19, the first retaining portion 21a and the second retaining portion 21b may be made of any appropriate material which will facilitate a connection that transmits compressive and tensile forces while providing for the aforementioned slidable motion in a generally transverse direction between each of the adjacent surfaces. For example, in the first embodiment, the first retaining portion 21a and the second retaining portion 21b may be typically made from any metal or metal alloy suitable for surgical implants such as stainless steel, titanium, and cobalt chromium, or composite materials such as carbon fiber, or a plastic material such as polyetheretherketone (PEEK) or any other suitable materials. The disc may be made from plastic such as high molecular weight polyethylene or PEEK, or from ceramics, metal, and natural or synthetic fibers such as, but not limited to, carbon fiber, rubber, or other suitable materials. Generally, to help maintain the sliding characteristic of the surfaces, the surfaces may be polished and/or coated to provide smooth surfaces. For example, if the surfaces are made of metal, the metal surfaces may be polished metal.

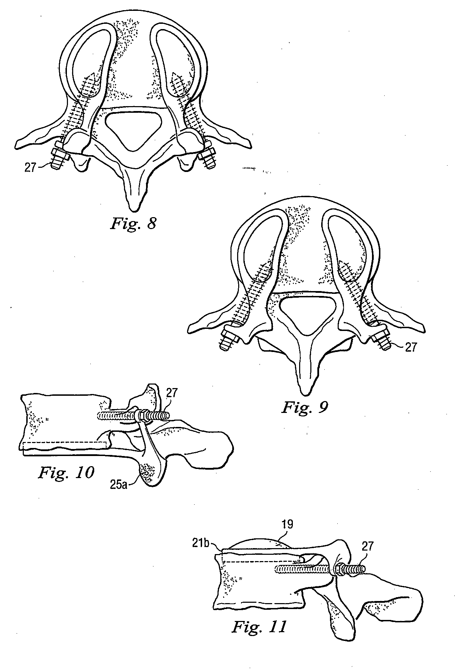

Figures 8-14 illustrate a second embodiment of an artificial intervertebral joint. Only features that differ from the first embodiment are discussed in detail herein. In the second exemplary embodiment, securing components, such as, for example, pedicle screws 27 are provided to provide a more secure and immediate connection between each of the first retaining portion 21a and/or the second retaining portion 21b to the corresponding vertebra. In addition, this embodiment illustrates a disc 19 which is integrated with one of the retaining portions, here lower retaining portion 21b. Disc 19 may be integrally formed from the same material as its retaining portion, but also may be separately formed from similar or dissimilar materials and permanently connected thereto to form an integral unit. In this embodiment, the disc 19 and the retaining portions may be all formed from metal.

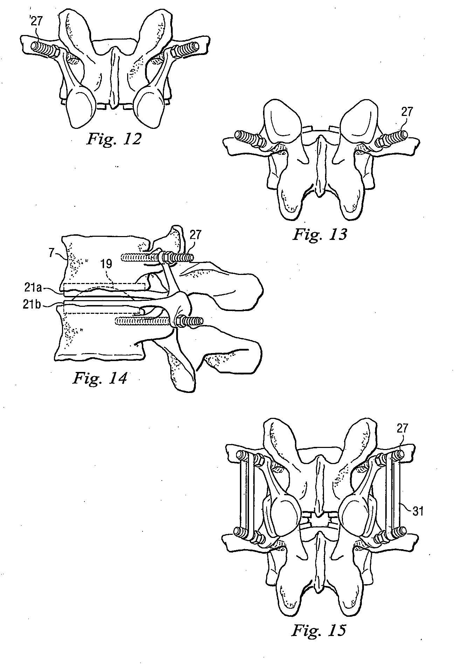

Figures 15 and 16 illustrate a third embodiment of an artificial intervertebral joint. In the third exemplary embodiment, additional securing components, such as, for example, tension bands 31 are provided to supplement or replace the function of posterior ligaments that limit the mobility between adjacent vertebrae by securing the first retaining portion 21a to the second retaining portion 21b. As shown in Figures 15-16, posterior tension bands 31 may be provided by wrapping them around the corresponding pedicle screws 27 or other convenient attachment points.

Figures 17 and 18 illustrate a fourth embodiment of an artificial intervertebral joint. In the exemplary embodiment illustrated in Figures 17 and 18, the artificial intervertebral joint may have all of the features discussed above except for artificial facet components. In this embodiment, the natural facet joints remain. The ligamentous tension band may also be left intact in some embodiments. In addition, this embodiment includes a specific example of an anterior midline connection between respective upper and lower retaining portions, which assists in maintaining the placement of the first retaining portion 21a and the second retaining portion 21b.

Figures 17 and 18 illustrate that it is possible to provide a first retaining portion 21a with a lock and key type pattern which is complemented by the corresponding mating portion provided on the second retaining portion 21b. More particularly, one half of the first retaining portion 21 a has an outer boundary with a U-shaped portion 35a while the other half of the corresponding first retaining portion 21 a has an outer boundary with a protruding portion 35b, which fits into the U-shaped portion 35a. As a result, each half of the first retaining portion 21a, 21b may be maintained in a predetermined position. However, the upper or lower retaining portions may fit together and/or be connected in the interbody space, e.g., near their midline anterior portions, in any manner that facilitates implantation and/or assists in providing and/or retaining the joint in a generally stable, symmetrical configuration. It may be even more important to provide such connection between the lower retaining portions due to the inward forces provided by annulus 17 remaining on the inferior end plate as shown in Figure 18. A midline connection between the respective lower retaining portions will resist the force of the outer annulus tending to cause migration of the retaining portions toward the midline 37.

As shown in the various exemplary embodiments, other than the portions of the first and/or second retaining portions which may fit together like a lock and key to maintain the placement of the portions relative to each other, each half of the artificial intervertebral joint may be generally symmetrical about the midline 37 of the vertebrae.

Again, these exemplary embodiments are merely illustrative and are not meant to be an exhaustive list of all possible designs, implementations, modifications, and uses of the invention. Moreover, features described in connection with one embodiment of the disclosure may be used in conjunction with other embodiments, even if not explicitly stated above.

While it should be readily apparent to a skilled artisan from the discussion above, a brief description of a suitable surgical procedure that may be used to implant the artificial joint is provided below. Generally, as discussed above, the artificial intervertebral joint may be implanted into a body using a posterior transforaminal approach similar to the known TLIF or PLIF procedures. According to this approach, an incision, such as a midline incision, may be made in the patient's back and some or all of the affected disc and surrounding tissue may be removed via the foramina. Depending on whether any of the facet joints are being replaced, the natural facet joints may be trimmed to make room for the artificial facet joints. Then, the halves of the artificial intervertebral joint may be inserted piecewise through the left and right transforaminal openings, respectively. That is, the pieces of the artificial intervertebral joint including the upper and lower retaining portions, with or without facet components, and the artificial disc, if provided separately, fit through the foramina and are placed in the appropriate intervertebral space. The pieces of the artificial joint may be completely separated or two or more of them may be tied or packaged together prior to insertion through the foramina by cloth or other materials known in the art. In cases where at least a portion of the outer annulus of the natural disc can be retained, the lower retaining portions of each side of the artificial intervertebral joint are inserted such that they abut a corresponding portion of the annulus. If a midline anterior connection is provided, the left and right halves of the retaining members are fitted together and held in place by the outer annulus. As such, the remaining portion of the annulus may be in substantially the same place as it was prior to the procedure.

Further, in the cases where the annulus of the natural disc must be removed completely or this is insufficient annulus remaining, it is possible, for example, to use the embodiment of the disclosure where the pedicle screws are implemented so as to be assured that the pieces of the artificial intervertebral joint remain in place. It should be understood by one of ordinary skill in the art that the artificial joint could be implanted via an anterior approach or a combined anterior and posterior approach, although the advantages of a posterior procedure would be limited. For example, some of the pieces of the artificial intervertebral joint may be inserted from an anterior approach and others posteriorally. The anterior and posteriorally placed portions could be fitted together similar to the embodiment shown in Figures 17 and 18.

Although only a few exemplary embodiments have been described in detail above, those skilled in the art will readily appreciate that many modifications are possible in the exemplary embodiments without materially departing from the novel teachings and advantages of this disclosure.

## Claims

1. An artificial spinal joint for creating at least a portion of a coupling between a superior vertebra (7) and an inferior vertebra (9) comprising:
an anterior joint replacement component (21a, 21b);
a bridge coupled to the anteriorjoint replacement component (21a, 21b) and extending posteriorly from the anterior joint replacement component (21a, 21b) beyond one or both generally cylindrical body portions of the superior (7) and inferior vertebrae (9); and
a posterior joint replacement component (23a, 23b) coupled to the bridge.

2. The artificial spinal joint of claim 1 wherein the coupling between the anterior joint replacement component (21a, 21b) and the bridge is a rigid coupling.

3. The artificial spinal joint of claim 1 wherein the coupling between the posterior joint replacement component (23a, 23b) and the bridge is a rigid coupling.

4. The artificial spinal joint of claim 1 wherein the artificial spinal joint includes a connection for accepting a fastener (27) for fixing the artificial spinal joint to the superior vertebra (7).

5. The artificial spinal joint of claim 4 wherein the connection for accepting a fastener (27) is integral with the anterior joint replacement component (21 a, 21b).

6. The artificial spinal joint of claim 4 wherein the connection for accepting a fastener (27) is integral with the posterior joint replacement component (23a, 23b).

7. The artificial spinal joint of claim 4 or 6 wherein the connection for accepting a fastener (27) is a connection for a pedicle screw (29).

8. The artificial spinal joint of claim 1 wherein the artificial spinal joint includes a connection for accepting a fastener (27) for fixing the artificial spinal joint to the inferior vertebra (7).

9. The artificial spinal joint of claim 8 wherein the connection for accepting a fastener (27) is integral with the anteriorjoint replacement component (21 a, 21b).

10. The artificial spinal joint of claim 8 wherein the connection for accepting a fastener (27) is integral with the posterior joint replacement component (23a, 23b).

11. The artificial spinal joint of claim 4 or 8 or 10 wherein the connection for accepting a fastener (27) is a connection for a pedicle screw (27).

12. The artificial spinal joint of claim 1 further comprising a pedicle screw (27).

13. The artificial spinal joint of claim 1 wherein a portion of a surface between the artificial spinal joint and the superior vertebra or the inferior vertebra includes material designed to promote bone ingrowth.

14. The artificial spinal joint of claim 13 wherein the material designed to promote bone ingrowth includes bead shaped material.

15. The artificial spinal joint of claim 13 wherein the material designed to promote bone ingrowth includes a plasma sprayed material.

16. The artificial spinal joint of claim 1 wherein the artificial spinal joint includes at least:
an upper member comprising
an upper anterior joint replacement component (21a),
an upper bridge coupled to the upper anterior joint replacement component (21a),
and an upper posteriorjoint replacement component (23a) coupled to the upper bridge; and
a lower member comprising
a lower anterior joint replacement component (21b),
a lower bridge coupled to the lower anterior joint replacement component (21b), and
a lower posterior joint replacement component (23b) coupled to the lower bridge.

17. The artificial spinal joint of claim 16 wherein the upper member is configured to be connected to the lower member.

18. The artificial spinal joint of claim 16 wherein the upper anterior joint replacement component (21a) is configured to be connected to the lower anterior joint replacement component (21b).

19. The artificial spinal joint of claim 16 wherein the upper posterior joint replacement component (23a) is configured to be connected to the lower posterior joint replacement component (23b).

20. The artificial spinal joint of claim 19 wherein the upper posterior joint replacement component (23b) is connected to the lower posterior joint replacement component (23b) with a tension band (31).

21. The artificial spinal joint of claim 1 wherein the bridge is at least a portion of an artificial pedicle.

## Patentansprüche

1. Ein künstliches Wirbelsäulengelenk zum Erzeugen von zumindest einem Abschnitt einer Verbindung zwischen einem oberen Wirbel (7) und einem unteren Wirbel (9), aufweisend:
eine anteriore Gelenk-Ersatzkomponente (21a, 21b),
eine Brücke, die mit der anterioren Gelenk-Ersatzkomponente (21a, 21b) verbunden ist und sich von der anterioren Gelenk-Ersatzkomponente (21a, 21b) aus posterior über einen oder beide im Wesentlichen zylinderförmigen Körperabschnitte des oberen (7) und des unteren Wirbels (9) hinaus erstreckt, und
eine posteriore Gelenk-Ersatzkomponente (23a, 23b), die mit der Brücke verbunden ist.

2. Das künstliche Wirbelsäulengelenk gemäß Anspruch 1, wobei die Verbindung zwischen der anterioren Gelenk-Ersatzkomponente (21a, 21b) und der Brücke eine steife Verbindung ist.

3. Das künstliche Wirbelsäulengelenk gemäß Anspruch 1, wobei die Verbindung zwischen der posterioren Gelenk-Ersatzkomponente (23a, 23b) und der Brücke eine steife Verbindung ist.

4. Das künstliche Wirbelsäulengelenk gemäß Anspruch 1, wobei das künstliche Wirbelsäulengelenk eine Verbindung zum Aufnehmen eines Befestigungselements (27) zum Fixieren des künstlichen Wirbelsäulengelenks an dem oberen Wirbel (7) aufweist.

5. Das künstliche Wirbelsäulengelenk gemäß Anspruch 4, wobei die Verbindung zum Aufnehmen eines Befestigungselements (27) mit der anterioren Gelenk-Ersatzkomponente (21a, 21b) einstückig ist.

6. Das künstliche Wirbelsäulengelenk gemäß Anspruch 4, wobei die Verbindung zum Aufnehmen eines Befestigungselements (27) mit der posterioren Gelenk-Ersatzkomponente (23a, 23b) einstückig ist.

7. Das künstliche Wirbelsäulengelenk gemäß Anspruch 4 oder 6, wobei die Verbindung zum Aufnehmen eines Befestigungselements (27) eine Verbindung für eine Pedikelschraube (29) ist.

8. Das künstliche Wirbelsäulengelenk gemäß Anspruch 1, wobei das künstliche Wirbelsäulengelenk eine Verbindung zum Aufnehmen eines Befestigungselements (27) zum Fixieren des künstlichen Wirbelsäulengelenks an dem unteren Wirbel (7) aufweist.

9. Das künstliche Wirbelsäulengelenk gemäß Anspruch 8, wobei die Verbindung zum Aufnehmen eines Befestigungselements (27) mit der anterioren Gelenk-Ersatzkomponente (21a, 21b) einstückig ist.

10. Das künstliche Wirbelsäulengelenk gemäß Anspruch 8, wobei die Verbindung zum Aufnehmen eines Befestigungselements (27) mit der posterioren Gelenk-Ersatzkomponente (23a, 23b) einstückig ist.

11. Das künstliche Wirbelsäulengelenk gemäß Anspruch 4 oder 8 oder 10, wobei die Verbindung zum Aufnehmen eines Befestigungselements (27) eine Verbindung für eine Pedikelschraube (27) ist.

12. Das künstliche Wirbelsäulengelenk gemäß Anspruch 1, das ferner eine Pedikelschraube (27) aufweist.

13. Das künstliche Wirbelsäulengelenk gemäß Anspruch 1, wobei ein Abschnitt einer Fläche zwischen dem künstlichen Wirbelsäulengelenk und dem oberen Wirbel oder dem unteren Wirbel Material aufweist, das zum Fördern von Knochen-Einwachsen eingerichtet ist.

14. Das künstliche Wirbelsäulengelenk gemäß Anspruch 13, wobei das zum Fördern von Knochen-Einwachsen eingerichtete Material wulstförmiges Material aufweist.

15. Das künstliche Wirbelsäulengelenk gemäß Anspruch 13, wobei das zum Knochen-Einwachsen eingerichtete Material ein Plasmaspritzmaterial aufweist.

16. Das künstliche Wirbelsäulengelenk gemäß Anspruch 1, wobei das künstliche Wirbelsäulengelenk mindestens aufweist:
ein oberes Element, das aufweist:
eine obere anteriore Gelenk-Ersatzkomponente (21a),
eine obere Brücke, die mit der oberen anterioren Gelenk-Ersatzkomponente (21a) verbunden ist,
und eine obere posteriore Gelenk-Ersatzkomponente (23a), die mit der oberen Brücke verbunden ist, und
ein unteres Element, das aufweist:
eine untere anteriore Gelenk-Ersatzkomponente (21b),
eine untere Brücke, die mit der unteren anterioren Gelenk-Ersatzkomponente (21b) verbunden ist, und
eine untere posteriore Gelenk-Ersatzkomponente (23b), die mit der unteren Brücke verbunden ist.

17. Das künstliche Wirbelsäulengelenk gemäß Anspruch 16, wobei das obere Element eingerichtet ist, um mit dem unteren Element verbunden zu sein.

18. Das künstliche Wirbelsäulengelenk gemäß Anspruch 16, wobei die obere anteriore Gelenk-Ersatzkomponente (21a) eingerichtet ist, um mit der unteren anterioren Gelenk-Ersatzkomponente (21b) verbunden zu sein.

19. Das künstliche Wirbelsäulengelenk gemäß Anspruch 16, wobei die obere posteriore Gelenk-Ersatzkomponente (23a) eingerichtet ist, um mit der unteren posterioren Gelenk-Ersatzkomponente (23b) verbunden zu sein.

20. Das künstliche Wirbelsäulengelenk gemäß Anspruch 19, wobei die obere posteriore Gelenk-Ersatzkomponente durch ein Spannungsband (31) mit der unteren posterioren Gelenk-Ersatzkomponente (23b) verbunden ist.

21. Das künstliche Wirbelsäulengelenk gemäß Anspruch 1, wobei die Brücke zumindest ein Abschnitt eines künstlichen Pedikels ist.

## Revendications

1. Articulation vertébrale artificielle pour créer au moins une partie d'un couplage entre une vertèbre supérieure (7) et une vertèbre inférieure (9), comprenant :
un composant de remplacement d'articulation antérieur (21a, 21b) ;
un pont couplé au composant de remplacement d'articulation antérieur (21a, 21b) et s'étendant en direction postérieure par rapport au composant de remplacement d'articulation antérieur (21a, 21b) au-delà d'une ou des deux parties de corps généralement cylindriques des vertèbres supérieure (7) et inférieure (9) ; et
un composant de remplacement d'articulation postérieur (23a, 23b) couplé au pont.

2. Articulation vertébrale artificielle selon la revendication 1, dans laquelle le couplage entre le composant de remplacement d'articulation antérieur (21a, 21b) et le pont est un couplage rigide.

3. Articulation vertébrale artificielle selon la revendication 1, dans laquelle le couplage entre le composant de remplacement d'articulation postérieur (23a, 23b) et le pont est un couplage rigide.

4. Articulation vertébrale artificielle selon la revendication 1, l'articulation vertébrale artificielle comprenant une connexion pour accepter un dispositif de fixation (27) afin de fixer l'articulation vertébrale artificielle à la vertèbre supérieure (7).

5. Articulation vertébrale artificielle selon la revendication 4, dans laquelle la connexion pour accepter un dispositif de fixation (27) est intégrée au composant de remplacement d'articulation antérieur (21a, 21b).

6. Articulation vertébrale artificielle selon la revendication 4, dans laquelle la connexion pour accepter un dispositif de fixation (27) est intégrée au composant de remplacement d'articulation postérieur (23a, 23b).

7. Articulation vertébrale artificielle selon la revendication 4 ou 6, dans laquelle la connexion pour accepter un dispositif de fixation (27) est une connexion pour une vis pédiculaire (29).

8. Articulation vertébrale artificielle selon la revendication 1, l'articulation vertébrale artificielle comprenant une connexion pour accepter un dispositif de fixation (27) afin de fixer l'articulation vertébrale artificielle à la vertèbre inférieure (7).

9. Articulation vertébrale artificielle selon la revendication 8, dans laquelle la connexion pour accepter un dispositif de fixation (27) est intégrée au composant de remplacement d'articulation antérieur (21a, 21b).

10. Articulation vertébrale artificielle selon la revendication 8, dans laquelle la connexion pour accepter un dispositif de fixation (27) est intégrée au composant de remplacement d'articulation postérieur (23a, 23b).

11. Articulation vertébrale artificielle selon la revendication 4 ou 8 ou 10, dans laquelle la connexion pour accepter un dispositif de fixation (27) est une connexion pour une vis pédiculaire (27).

12. Articulation vertébrale artificielle selon la revendication 1, comprenant en outre une vis pédiculaire (27).

13. Articulation vertébrale artificielle selon la revendication 1, dans laquelle une partie d'une surface entre l'articulation vertébrale artificielle et la vertèbre supérieure ou la vertèbre inférieure comprend un matériau conçu pour favoriser la repousse osseuse.

14. Articulation vertébrale artificielle selon la revendication 13, dans laquelle le matériau conçu pour favoriser la repousse osseuse comprend un matériau en forme de bille.

15. Articulation vertébrale artificielle selon la revendication 13, dans laquelle le matériau conçu pour favoriser la repousse osseuse comprend un matériau projeté par plasma.

16. Articulation vertébrale artificielle selon la revendication 1, l'articulation vertébrale artificielle comprenant au moins :
un élément supérieur comprenant
un composant de remplacement d'articulation antérieur supérieur (21a),
un pont supérieur couplé au composant de remplacement d'articulation antérieur supérieur (21a),
et un composant de remplacement d'articulation postérieur supérieur (23a) couplé au pont supérieur ; et
un élément inférieur comprenant
un composant de remplacement d'articulation antérieur inférieur (21b),
un pont inférieur couplé au composant de remplacement d'articulation antérieur inférieur (21b), et
un composant de remplacement d'articulation postérieur inférieur (23b) couplé au pont inférieur.

17. Articulation vertébrale artificielle selon la revendication 16, dans laquelle l'élément supérieur est configuré pour être connecté à l'élément inférieur.

18. Articulation vertébrale artificielle selon la revendication 16, dans laquelle le composant de remplacement d'articulation antérieur supérieur (21a) est configuré pour être connecté au composant de remplacement d'articulation antérieur inférieur (21b).

19. Articulation vertébrale artificielle selon la revendication 16, dans laquelle le composant de remplacement d'articulation postérieur supérieur (23a) est configuré pour être connecté au composant de remplacement d'articulation postérieur inférieur (23b).

20. Articulation vertébrale artificielle selon la revendication 19, dans laquelle le composant de remplacement d'articulation postérieur supérieur (23b) est connecté au composant de remplacement d'articulation postérieur inférieur (23b) avec une bande de tension (31).

21. Articulation vertébrale artificielle selon la revendication 1, dans laquelle le pont est au moins une partie d'un pédicule artificiel.
